# EUROPÄISCHE PATENTSCHRIFT

(11) **EP 1 316 545 B1**
(45) Veröffentlichungstag und Bekanntmachung des Hinweises auf die Patenterteilung: **08.12.2004**
(21) Anmeldenummer: 02022627.0
(22) Anmeldetag: 09.10.2002
(51) Int. Cl.: C07C 249/04

(54) **Zweiphasige Ammoximierung**
Biphasic ammoximation
Ammoximation biphasique

(30) Priorität: 28.11.2001 DE 10158352
(43) Veröffentlichungstag der Anmeldung: 04.06.2003
(73) Patentinhaber: Degussa AG, 40474 Düsseldorf (DE)
(72) Erfinder: Herwig, Jürgen, Dr., 46569 Hünxe (DE); Leininger, Stefan, Dr., 63452 Hanau (DE); Oenbrink, Georg, Dr., 48249 Dülmen (DE); Schiffer, Thomas, Dr., 45721 Haltern (DE)

(56) Entgegenhaltungen:
- EP-A- 0 267 362
- EP-A- 0 496 385
- EP-A- 1 227 080

## Beschreibung

Die Erfindung betrifft ein Verfahren zur Herstellung von Oximen aus Ketonen oder Aldehyden. Dabei wird das Keton oder der Aldehyd in einem unter den Reaktionsbedingungen inerten Kohlenwasserstoff gelöst und mit Ammoniak und Wasserstoffperoxid in einem System mit zwei flüssigen Phasen in Gegenwart eines Phasenvermittlers an einem heterogenen Katalysatorsystem umgesetzt, wobei das Katalysatorsystem aus zwei oder mehr Komponenten besteht, wovon mindestens eine aus einem Titansilikalit und eine andere aus einem homogen gelösten Ammoniumsalz besteht.

In der europäischen Patentanmeldung EP-A-0 267 362 wird in den Beispielen 8 und 9 die zweiphasige Herstellung von Cyclohexanonoxim erwähnt. Es wird Toluol als Lösemittel verwendet, das jedoch den Nachteil hat, dass es nicht inert gegen konzentrierte Schwefelsäure ist. Dieses ist von entscheidender Bedeutung, da bei hochsiedenden Oximen wie zum Beispiel Cyclododecanonoxim das nach der Reaktion vorliegende Oxim mit Schwefelsäure aus einem Lösemittel extrahiert wird. Bei Verwendung von Toluol als Lösemittel der Ammoximierung muss also zuerst ein Lösemittelaustausch stattfinden, da Toluol nicht inert gegenüber Schwefelsäure ist. Dieses bedeutet eine zusätzliche Verfahrensstufe. Zudem wird in EP-A-0 267 362 in Beispiel 8 im zweiphasigen System nur ein Umsatz < 90 % erzielt. Hohe Umsätze sind für einen technischen Einsatz bei größeren Ringen wie zum Beispiel bei Cyclododecanon aber sehr wichtig, da sich mit wachsendem Molekulargewicht das nicht umgesetzte Keton vom entsprechenden Oxim nur unter großem technischen Aufwand abtrennen lässt. In EP-A-0 267 362, Beispiel 9, wird das ternäre Lösemittelgemisch Toluol, tert.-Butanol und Wasser zur Herstellung von Cyclohexanonoxim verwendet. Dieses ternäre Lösemittelgemisch hat den Nachteil, dass sich das nach der Reaktion vorhandene Oxim auf beide Phasen verteilt und damit eine vollständige Abtrennung des Oxims durch Phasentrennung nicht möglich ist. Außerdem sind die in EP-A-0 267 362, Beispiel 8 und 9, erzielten Umsatzraten mit 1,1 g Oxim/g Katalysator * h klein. Es wird kein Beispiel zur zweiphasigen Ammoximierung von Cyclododecanon offengelegt.

Eni-Chem beansprucht in der deutschen Offenlegungsschrift DE 195 21 011 A1 (entspricht US 5 498 793) ein Verfahren zur Ammoximierung von Acetophenon und Cyclododecanon. Hierbei wird auch die Verwendung von C₅ - C₈ aliphatischen Kohlenwasserstoffen als Lösemittel beansprucht, ohne ein Verfahrensbeispiel für eine solche Reaktion offenzulegen.

Es bestand daher die Aufgabe, ein Verfahren zur Ammoximierung von Ketonen und Aldehyden, insbesondere von vergleichsweise großen und sperrigen Ketonen wie Acetophenon und Cyclododecanon, bereit zu stellen, bei dem die Produktabtrennung vollständig über eine Phasentrennung erfolgen kann, der Umsatz bei einer Peroxidausbeute von > 50 % möglichst vollständig ist, die Umsatzrate in einem technisch akzeptablen Bereich liegt und das verwendete Lösemittel inert gegenüber Schwefelsäure ist. Der Umsatz sollte dabei nach Möglichkeit so hoch sein, dass auf eine Nachreaktion mit einer wässrigen Hydroxylaminlösung, wie sie von Eni-Chem in der europäischen Patentanmeldung EP-A-0 564 040 am Beispiel Cyclohexanon beschrieben wird, verzichtet werden kann.

Überraschend wurde nun gefunden, dass sich Ketone und Aldehyde in Gegenwart von unter den Reaktionsbedingungen inerten Kohlenwasserstoffen mit hoher Umsatzrate und Peroxidausbeute mit einem Titansilikalit als heterogenem Katalysator ammoximieren lassen, wenn Ammoniumsalze als homogener oder suspendierter Cokatalysator und ein oder mehrere Phasenvermittler zugesetzt werden.

Die vorliegende Erfindung betrifft daher ein Verfahren zur Herstellung von Oximen durch Umsetzung von Ketonen oder Aldehyden mit Wasserstoffperoxid und Ammoniak in einem System aus zwei flüssigen Phasen, wobei die eine Phase ein wässriges System ist und die andere Phase mindestens einen unter den Reaktionsbedingungen inerten Kohlenwasserstoff enthält, in Gegenwart eines Katalysatorsystems, welches aus mindestens zwei Komponenten besteht, wobei eine Komponente auf Basis von Titan, Silizium und Sauerstoff, vorzugsweise in Form eines Titansilikalits, aufgebaut ist, und als zweite Komponente ein Ammoniumsalz eingesetzt wird, das vorzugsweise homogen gelöst vorliegt oder bei hohen Konzentrationen auch teilweise suspendiert ist, und ein oder mehrere Tenside oder eine Mischung aus einem oder mehreren Tensiden und einem oder mehreren Phasentransferkatalysatoren als Phasenvermittler anwesend sind. Aus praktischen Gründen ist die Anzahl der Phasenvermittlerkomponenten jeweils maximal 3, vorzugsweise 1.

Der Katalysator ist auf Basis von Titan, Silizium und Sauerstoff aufgebaut. Der Katalysator ist vorzugsweise ein Titansilikalit, welches zum Beispiel als Titansilikalit TS1 kommerziell zur Verfügung steht.

Der Katalysator kann als Feststoff sowohl kristallin als Pulver als auch als Formkörper verwendet werden. Wird der Katalysator als Formkörper eingesetzt, kann neben der Titan/Silizium/Sauerstoff-Komponente mindestens eine weitere Komponente aus einem sauren Feststoff, der ein anorganisches oder organisches Trägermaterial enthält, anwesend sein, wobei entweder das Trägermaterial selbst lewis- oder brönstedsaure Eigenschaften besitzt oder entsprechende lewis- oder brönstedsaure funktionelle Gruppen auf das Trägermaterial aufgebracht werden und die Einbringung solcher Gruppen physikalisch oder chemisch erfolgt. Das Trägermaterial kann gleichzeitig auch als Binder des Formkörpers fungieren. Trägermaterial ist zum Beispiel ein saurer, anorganischer Feststoff auf Basis von Aluminiumoxid oder Alumosilikat. Als Trägermaterial kann aber auch ein organischer Feststoff auf Basis saurer oder stark saurer Ionenaustauscher verwendet werden.
Das Gewichtsverhältnis Katalysator zu Trägermaterial, wenn verwendet, beträgt vorzugsweise 0,1 : 1 bis 10 : 1.
Der Katalysator wird in Mengen von 0,2 - 5 Gewichtsprozent, bezogen auf die gesamte Reaktionslösung, eingesetzt.
Der Katalysator kann auch in Form eines festen Bettes angeordnet sein (Festbettkatalysator), über den die Reaktionsmischung geleitet wird. Die Verweilzeit im Festbett beträgt dabei vorzugsweise 0,1 bis 120 Sekunden, besonders bevorzugt 0,5 bis 60 Sekunden.

Als homogener Cokatalysator für das erfindungsgemäße Verfahren können alle Ammoniumsalze eingesetzt werden, die in der Reaktionsmischung ausreichend löslich sind und deren Anionen sich nicht nachteilig auf den Verlauf der Reaktion auswirken. Nicht limitierende Beispiele sind Ammoniumsalze von starken Mineralsäuren, wie zum Beispiel Ammoniumchlorid, Ammoniumsulfat oder Ammoniumnitrat, sowie Ammoniumsalze von Carbonsäuren, wie zum Beispiel Ammonium-formiat, -acetat, -propionat, -oxalat, -glutyrat, -citrat oder -benzoat. Die Menge des Ammoniumsalzes kann in weiten Grenzen gewählt werden. Bevorzugt wird das Ammoniumsalz in einer Konzentration von 0,001 mol/kg bis 1 mol/kg eingesetzt. Das Ammoniumsalz wird vorzugsweise entweder direkt der Reaktionsmischung oder dem zur Reaktion eingesetzten Wasserstoffperoxid zugegeben.

In einer weiteren Ausführungsform der Erfindung wird das Ammoniumsalz in der Reaktionsmischung aus einer Brönsted-Säure und dem zur Reaktion eingesetzten Ammoniak erzeugt. Nicht limitierende Beispiele für geeignete Brönsted-Säuren sind Mineralsäuren, wie zum Beispiel Salzsäure, Schwefelsäure und Salpetersäure, und Carbonsäuren, wie zum Beispiel Ameisensäure, Essigsäure, Propionsäure, Oxalsäure, Glutarsäure, Citronensäure oder Benzoesäure. Die Brönsted-Säure wird dabei vorzugsweise entweder direkt der Reaktionsmischung oder dem zur Reaktion eingesetzten Wasserstoffperoxid zugegeben. Der Cokatalysator verbleibt nach der Reaktion in der wässrigen Phase.

Als Phasenvermittler werden Tenside gegebenenfalls in Kombination mit sogenannten Phasentransferkatalysatoren eingesetzt, soweit sie stabil sind, also nicht in situ oxidiert werden. Als Beispiele für Tenside sei Alkansulfonat, insbesondere das Natriumsalz der Alkansulfonsäure mit 13 bis 17 Kohlenstoffatomen in der linearen oder verzweigten, vorzugsweise linearen, Kohlenstoffkette (wie zum Beispiel Marlon PS 30 der Sasol GmbH) oder das Natriumsalz der Alkylbenzolsulfonsäure mit 10 bis 13 Kohlenstoffatomen in der linearen oder verzweigten Kohlenstoffkette (wie zum Beispiel Marlon A 315 der Sasol GmbH) genannt, ohne die Erfindung auf diese Tenside zu beschränken. Es können auch Mischungen von Tensiden eingesetzt werden.

Weitere mögliche Phasenvermittler, die gegebenfalls zusätzlich zu dem Tensid eingesetzt werden können, sind quartäre Ammoniumsalze des Typs NR₁R₂R₃R₄⁺ X⁻ , wobei die Reste R₁ - R₄ unabhängig voneinander vorzugsweise aliphatische Kohlenwasserstoffreste von C₁ - C₂₀ sein können und X⁻ für ein Anion wie zum Beispiel Chlorid, Bromid, lodid oder Hydrogensulfat, steht. Beispiele sind Tetrabutylammonium- und Benzyltrimethylammonium-Salze. Weitere mögliche zusätzliche Phasenvermittler sind Phosphonium-Salze wie zum Beispiel Tetrabutylphosphonium-Salze, Onium-Verbindungen, Kronenether (vor allem 18-Krone-6) und Polyethylenglykole. Die Phasenvermittler werden in Konzentrationen von 0,01 Gew.-% - 5 Gew.-%, bezogen auf die gesamte Reaktionsmischung, zugesetzt.

Die Reaktion verläuft bezüglich der Ammoximierung der Carbonylverbindung hochgradig selektiv. Auch bei hohen Umsätzen (> 99 %) liegt die Selektivität des Oxims laut GC-Analyse bei über 99 %. Wird zum Beispiel technisch reines Cyclododecanon eingesetzt, lassen sich als Nebenprodukte im GC lediglich Spuren von Cyclododecan und Cyclododecanol nachweisen, welche bereits im Cyclododecanon als Verunreinigung vorhanden waren. Als weiteres Nebenprodukt wird in wenigen Fällen Laurinlactam in Konzentrationen < 0,1 % und das aus der reversiblen Reaktion mit Ammoniak gebildete Ketimin Cyclododecanonimin in Konzentrationen < 0,5 % nachgewiesen.

Als zweite Phase bildende Lösemittel dienen vorzugsweise Kohlenwasserstoffe, die gegenüber Wasserstoffperoxid und Ammoniak und konzentrierter Schwefelsäure stabil sind und sowohl für die Carbonylverbindung als auch für das korrespondierende Oxim eine ausreichende Löslichkeit aufweisen. Vorzugsweise eignen sich als Lösemittel C₆ - C₁₂ aliphatische und cycloaliphatische Kohlenwasserstoffe. Besonders geeignet für die Umsetzung von zum Beispiel Cyclododecanon sind Isopropylcyclohexan, Cyclooctan und Cyclododecan, ohne die Erfindung auf diese zu beschränken.

Wasserstoffperoxid wird als wässrige Lösung in handelsüblichen Konzentrationen, bevorzugt mindestens 30 gew.-%ig, eingesetzt. Ammoniak wird entweder als konzentrierte, wässrige Lösung (> 20 %ig) oder bevorzugt als Gas dem Reaktor zugeführt. Vorteile ergeben sich bei der gasförmigen Dosierung des Ammoniaks und bei hoch konzentrierten Peroxidlösungen aus der geringeren Menge an Wasser, die bei der Aufarbeitung der Reaktionsmischung vom homogenen Katalysator abgetrennt werden muss.

Die Reaktionstemperatur der Ammoximierung liegt zwischen 20 °C und 150 °C, bevorzugt zwischen 50 °C und 120 °C und besonders bevorzugt zwischen 60 °C und 100 °C. Der Reaktor wird dabei entweder bei Norinaldruck, das heißt dem Dampfdruck des jeweiligen Lösemittels bei der Reaktionstemperatur oder bei einem leichten Überdruck, vorzugsweise zwischen 1 bar und 10 bar betrieben. Der Überdruck kann mit Ammoniak oder einem Inertgas eingestellt werden. Wird der Reaktor verschlossen, steigt der Druck durch Bildung von gasförmigen Zersetzungsprodukten in Nebenreaktionen (vor allem Stickstoff und Sauerstoff) während der Reaktion an. Vorteilhaft ist es, den Reaktor isobar zu fahren, indem gasförmige Zersetzungsprodukte über einen leichten Abgasstrom mit Blasenzähler kontrolliert entweichen können und gegebenenfalls verbrauchter Ammoniak nachgedrückt wird.

Bei der Ammoximierungsreaktion können Carbonylverbindung und Wasserstoffperoxid jeweils diskontinuierlich oder kontinuierlich zudosiert werden. Da Zersetzungsreaktionen des H₂O₂ immer nebenbei auftreten, wird für einen vollständigen Umsatz von zum Beispiel Cyclododecanon ein Überschuss an Peroxidlösung benötigt, welcher durch geeignete Reaktionsführung und die erfindungsgemäßen Katalysatorsysteme minimiert werden kann. Bei den Versuchen hat es sich als vorteilhaft erwiesen, zu Beginn der Reaktion entweder die Carbonylverbindung vorzulegen oder sie in äquimolaren Mengen parallel zum Wasserstoffperoxid zu dosieren und den benötigten Überschuss an Peroxid nach beendeter Carbonylzugabe gemäß dem Verbrauch nachzudosieren.

Als Ketone und Aldehyde werden vorzugsweise große und sperrige Carbonylverbindungen mit 8 bis 20 Kohlenstoffatomen eingesetzt. Als Beispiele seien Acetophenon und die cyclischen Ketone Cyclooctanon, Cyclodecanon, Cyclododecanon und Cyclopentadecanon genannt. Prinzipiell sind jedoch alle Ketone geeignet, die bei der zweiphasigen Ammoximierung vorwiegend in der organischen Phase gelöst sind.

### Beispiele:

Um exakt gleiche Reaktionsbedingungen zu gewähren, wurde bei allen Beispielen jeweils frischer Katalysator (Titansilikalit TS1, Degussa AG) der selben Charge eingesetzt. Eine zusätzliche Aktivierung des Katalysators vor der Reaktion erfolgte nicht. Der Katalysator wurde nach der Reaktion bei 75 °C über einen Druckfilter abgetrennt und so zurückgewonnen.

### Beispiel 1:

In einem mit Stickstoff gespülten, beheizbaren 1,6 l Druckreaktor aus Glas (Büchi) mit Magnetkupplung, Gaseinleitungsrührer (500 U/Min), Purge und Druckregler werden bei 40 °C 91,2 g (500 mmol) Cyclododecanon in 274 g Isoproyplcyclohexan vorgelegt. 5,0 g Katalysator (TS1, Degussa AG), 73 g Wasser, 2,55 g Alkansulfonat (Marlon PS 30 der Sasol GmbH) und 8,28 g Citronensäure als Cokatalysatorbaustein zugegeben. Der Reaktor wird auf 95 °C erwärmt und auf 0,1 bar (Überdruck) entspannt, anschließend wird langsam Ammoniakgas bis auf einen Druck von 1,6 bar aufgedrückt. Dabei werden ca. 20 g Ammoniak zugegeben.

Während der Reaktion wird der Druck über einen leichten Abgasstrom konstant gehalten, gegebenfalls wird Ammoniakgas nachdosiert. Über einen Zeitraum von 180 Minuten werden 37,0 ml einer 50 gew.-%igen Wasserstoffperoxid-Lösung (entspricht 650 mmol H₂O₂) über eine Pumpe zudosiert. Nach beendeter Peroxidzugabe lässt man die Reaktionsmischung noch 60 Minuten nachreagieren.
Der Umsatz wird während der Reaktion per Gaschromatographie (GC) verfolgt und Wasserstoffperoxid iodometrisch bestimmt. Nach 240 Minuten beträgt der Umsatz 97,2 %, dies entspricht einer Peroxidselektivität von 74,7 %.

### Beispiel 2:

Der Versuch wird entsprechend Beispiel 1 wiederholt. Die Dosierzeit beträgt 300 Minuten und die Nachreaktionszeit 60 Minuten. Nach 360 Minuten beträgt der Umsatz 99,4 %. Es werden 1,44 Äquivalente. H₂O₂ verbraucht, dies entspricht einer Peroxidselektivität von 69,1 %.

### Beispiel 3 (Vergleichsbeispiel):

Der Versuch wird entsprechend Beispiel 2 wiederholt. Es werden bei 40 °C 54,7 g (300 mmol) Cyclododecanon in 310 g Isopropylcyclohexan vorgelegt. 2,5 g Katalysator (TS1, Degussa AG), 73 g Wasser und 2,55 g Alkansulfonat (Marlon PS 30 der Sasol GmbH) werden zugegeben. Nach 360 Minuten beträgt der Umsatz 20,0 %, es werden 2,40 Äquivalente H₂O₂ verbraucht, dies entspricht einer Peroxidselektivität von 8,4 %. Es zeigt sich, dass ohne Cokatalysator nur sehr schlechte Umsätze und Selektivitäten erreicht werden.

### Beispiel 4 (Vergleichsbeispiel):

In einem mit Stickstoff gespülten, beheizbaren 100 ml Doppelmantelreaktor aus Glas mit Gaseinleitungsrührer (1500 U/Min), Purge und Druckregler werden bei 60 °C 50 mmol Cyclododecanon in 50 ml Isopropylcyclohexan vorgelegt. 1,0 g Katalysator (TS 1, Degussa AG) werden zugegeben. Der Reaktor wird auf 60 °C erwärmt, anschließend wird langsam Ammoniakgas bis auf einen Druck von 1,1 bar aufgedrückt.
Während der Reaktion wird der Druck über einen leichten Abgasstrom konstant gehalten, gegebenfalls wird Ammoniakgas nachdosiert. Über einen Zeitraum von 180 Minuten werden 100 mmol einer 50 gew.-%igen Wasserstoffperoxid-Lösung mit eine Pumpe zudosiert. Nach beendeter Peroxidzugabe lässt man die Reaktionsmischung noch 120 Minuten nachreagieren. Der Umsatz wird während der Reaktion per GC verfolgt und Wasserstoffperoxid iodometrisch bestimmt. Nach 300 Minuten beträgt der Umsatz 2,9 %, dies entspricht einer Peroxidselektivität von 1,4 %. Es zeigt sich, dass ohne Cokatalysator und ohne Phasenvermittler nur sehr schlechte Umsätze und Selektivitäten erreicht werden.

### Beispiele 5 - 10:

Der Versuch entsprechend Beispiel 3 wird wiederholt, nur dass die Dosierzeit des H₂O₂ 240 Minuten und die Nachreaktionszeit 60 Minuten beträgt und verschiedene Ammoniumsalze zugefügt werden. Alle Salze werden in der gleichen Konzentration von 0,1 mol/l bezogen auf das gesamte zweiphasige Gemisch zugegeben. Die Ergebnisse sind in Tabelle 1 zusammengefasst.

**Tabelle 1:**

| **Beispiel Nr.** | **Ammoniumsalz** | **Umsatz [%]** | **H**_{**2**}**O**_{**2**}**-Selektivität** |
|---|---|---|---|
| | | | |
| 3 | ohne | 20 | 8 |
| 5 | Acetat | 77 | 39 |
| 6 | Citrat | 90 | 45 |
| 7 | Glutyrat | 66 | 33 |
| 8 | Benzoat | 89 | 44 |
| 9 | Phosphat | 40 | 20 |
| 10 | Sulfat | 68 | 34 |

## Patentansprüche

1. Verfahren zur Herstellung von Oximen durch Umsetzung von Ketonen oder Aldehyden mit Wasserstoffperoxid und Ammoniak,
**dadurch gekennzeichnet,**
**dass** die Umsetzung in einem System aus einer wässrigen und aus einer unter den Reaktionsbedingungen inerten Kohlenwasserstoffen bestehenden Phase in Gegenwart eines Katalysatorsystems, welches aus mindestens zwei Komponenten besteht, durchgeführt wird und wobei eine Komponente des Katalysatorsystems heterogen vorliegt und auf Basis von Titan, Silizium und Sauerstoff aufgebaut ist und die zweite Komponente ein homogen gelöstes oder suspendiertes Ammoniumsalz ist, und wobei mindestens ein Phasenvermittler anwesend ist.

2. Verfahren nach Anspruch 1,
**dadurch gekennzeichnet,**
**dass** als Phasenvermittler ein Tensid, ein Gemisch von Tensiden oder ein Gemisch aus einem Tensid und einem Phasentransferkatalysator eingesetzt wird.

3. Verfahren nach Anspruch 2,
**dadurch gekennzeichnet,**
**dass** das Tensid beziehungsweise eines der Tenside ein Alkansulfonat ist.

4. Verfahren nach einem der vorhergehenden Ansprüche,
**dadurch gekennzeichnet,**
**dass** die Titan/Silizium/Sauerstoff-Komponente ein Titansilikalit ist.

5. Verfahren nach Anspruch 4,
**dadurch gekennzeichnet,**
**dass** die Titan/Silizium/Sauerstoff-Komponente der Titansilikalit TS1 ist.

6. Verfahren nach Anspruch 1,
**dadurch gekennzeichnet,**
**dass** neben der Titan/Silizium/Sauerstoff-Komponente mindestens eine weitere Komponente aus einem sauren Feststoff, der ein anorganisches oder organisches Trägermaterial enthält, anwesend ist, wobei entweder das Trägermaterial selbst lewis- oder brönstedsaure Eigenschaften besitzt oder entsprechende lewis- oder brönstedsaure funktionelle Gruppen auf das Trägermaterial aufgebracht werden und die Einbringung solcher Gruppen physikalisch oder chemisch erfolgt.

7. Verfahren nach Anspruch 6,
**dadurch gekennzeichnet,**
**dass** als Trägermaterial ein saurer, anorganischer Feststoff auf Basis von Aluminiumoxid oder Alumosilikat verwendet wird.

8. Verfahren nach einem der vorhergehenden Ansprüche,
**dadurch gekennzeichnet,**
**dass** als Trägermaterial ein organischer Feststoff auf Basis saurer oder stark saurer Ionenaustauscher verwendet wird.

9. Verfahren nach einem der vorhergehenden Ansprüche,
**dadurch gekennzeichnet,**
**dass** das Gewichtsverhältnis Katalysator zu Trägermaterial 0,1 : 1 bis 10 : 1 beträgt.

10. Verfahren nach einem der vorhergehenden Ansprüche,
**dadurch gekennzeichnet,**
**dass** Katalysator oder Katalysator und Trägermaterial in Pulverform eingesetzt werden.

11. Verfahren nach einem der Ansprüche 1 bis 9,
**dadurch gekennzeichnet,**
**dass** Katalysator und Trägermaterial als Formkörper eingesetzt werden.

12. Verfahren nach einem der vorhergehenden Ansprüche,
**dadurch gekennzeichnet,**
**dass** ein Formkörper eingesetzt wird, bei dem das Trägermaterial gleichzeitig auch als Binder des Formkörpers fungiert.

13. Verfahren nach einem der vorherigen Ansprüche,
**dadurch gekennzeichnet,**
**dass** der heterogene Katalysator in Form eines Festbetts vorliegt.

14. Verfahren nach einem der vorherigen Ansprüche,
**dadurch gekennzeichnet,**
**dass** das Anion des Ammoniumsalzes ein Halogenid-, Sulfat- Hydrogensulfat-, Nitrat- oder Phosphat-Ion oder ein Anion einer Carbonsäure ist.

15. Verfahren nach Anspruch 13,
**dadurch gekennzeichnet,**
**dass** als Ammoniumsalz Ammonium-formiat, -acetat, -propionat, -oxalat, -glutyrat, - malonat, -citrat oder -benzoat anwesend ist.

16. Verfahren nach einem der vorherigen Ansprüche,
**dadurch gekennzeichnet,**
**dass** die Reaktionstemperatur zwischen 20 °C und 150 °C liegt.

17. Verfahren nach Anspruch 16,
**dadurch gekennzeichnet,**
**dass** die Reaktionstemperatur zwischen 60 °C und 100 °C liegt.

18. Verfahren nach einem der vorhergehenden Ansprüche,
**dadurch gekennzeichnet,**
**dass** der Druck während der Reaktion 1 bar bis 10 bar beträgt.

19. Verfahren nach einem der vorhergehenden Ansprüche,
**dadurch gekennzeichnet,**
**dass** als Carbonylverbindung Acetophenon oder cyclische Ketone mit 8 bis 20 Kohlenstoffatomen ammoximiert werden.

20. Verfahren nach Anspruch 18,
**dadurch gekennzeichnet,**
**dass** Cyclododecanon ammoximiert wird.

## Claims

1. A process for preparing an oxime by reacting a ketone or aldehyde with hydrogen peroxide and ammonia, **characterized in that** the reaction is carried out in a system of an aqueous phase and a hydrocarbon phase inert under the reaction conditions, in the presence of a catalyst system which consists of at least two components, one component of the catalyst system being present in heterogeneous form and being based on titanium, silicon and oxygen and a second component being a homogeneously dissolved or suspended ammonium salt, and at least one interphase contactor being present.

2. A process according to claim 1, **characterized in that** the interphase contactor is a surfactant, a mixture of surfactants or a mixture of a surfactant and a phase-transfer catalyst.

3. A process according to claim 2, **characterized in that** the surfactant or one of the surfactants is an alkane sulphonate.

4. A process according to any one of the preceding claims, **characterized in that** the titanium/silicon/oxygen component is a titanium silicalite.

5. A process according to claim 4, **characterized in that** the titanium/silicon/oxygen component is the titanium silicalite TS1.

6. A process according to claim 1, **characterized in that**, in addition to the titanium/silicon/oxygen componert, at least one further component comprising an acidic solid which contains an inorganic or organic support material is present, either the support material itself having Lewis acid or Brönsted acid properties or corresponding Lewis acid or Brönsted acid functional groups being applied to the support material, such groups being introduced physically or chemically.

7. A process according to claim 6, **characterized in that** the support material is an acidic inorganic solid based on aluminium oxide or aluminosilicate.

8. A process according to any one of the preceding claims, **characterized in that** the support material is an organic solid based on acidic or strongly acidic ion exchanger.

9. A process according to any one of the preceding claims, **characterized in that** the weight ratio of catalyst to support material is from 0.1:1 to 10:1.

10. A process according to any one of the preceding claims, **characterized in that** catalyst or catalyst and support material are in powder form.

11. A process according to any one of claims 1 to 9, **characterized in that** catalyst and support material are used as shaped bodies.

12. A process according to any one of the preceding claims, **characterized in that** a shaped body is used in which the support material simultaneously also acts as the binder to the shaped body.

13. A process according to any one of the preceding claims, **characterized in that** the heterogeneous catalyst is present in the form of a fixed bed.

14. A process according to any one of the preceding claims, **characterized in that** the anion of the ammonium salt is a halide, sulphate, hydrogensulphate, nitrate or phosphate ion or an anion of a carboxylic acid.

15. A process according to claim 13, **characterized in that** the ammonium salt is present as ammonium formate, acetate, propionate, oxalate, glutarate, malonate, citrate or benzoate.

16. A process according to any one of the preceding claims, **characterized in that** the reaction temperature is between 20°C and 150°C.

17. A process according to claim 16, **characterized in that** the reaction temperature is between 60°C and 100°C.

18. A process according to any one of the preceding claims, **characterized in that** the pressure during the reaction is from 1 bar to 10 bar.

19. A process according to any one of the preceding claims, **characterized in that** the carbonyl compound ammoximated is acetophenone or a cyclic ketone having from 8 to 20 carbon atoms.

20. A process according to claim 18, **characterized in that** cyclododecanone is ammoximated.

## Revendications

1. Procédé pour la fabrication d'oximes par la conversion de cétones ou d'aldéhydes avec du peroxyde d'hydrogène et de l'ammonium,
**caractérisé en ce que**
la conversion est réalisée dans un système se composant d'une phase aqueuse et d'une phase existant dans des conditions de réaction des hydrocarbures inertes en présence d'un système de catalyseurs qui se compose d'au moins deux composants, et dans lequel un composant du système de catalyseurs est présent de façon hétérogène et est constitué à base de titane, de silicium ou d'oxygène, et le second composant est un sel d'ammonium suspendu ou dissous de façon homogène et dans lequel au moins un agent de transition de phase est présent.

2. Procédé selon la revendication 1,
**caractérisé en ce que**
comme agent de phase on utilise un agent tensioactif, un mélange d'agents tensioactifs, ou un mélange d'un agent tensioactif et d'un catalyseur de transfert de phase.

3. Procédé selon la revendication 2,
**caractérisé en ce que**
l'agent tensioactif, de préférence un des agents tensioactifs, est un alcane sulfonate.

4. Procédé selon l'une quelconque des revendications précédentes,
**caractérisé en ce que**
le composant titane/silicium/oxygène est une silicalite de titane.

5. Procédé selon la revendication 4,
**caractérisé en ce que**
le composant titane/silicium/oxygène est de la silicalite de titane TS1.

6. Procédé selon la revendication 1,
**caractérisé en ce que**
parallèlement au composant titane/silicium/oxygène, au moins un autre composant composé d'un solide acide qui contient un substrat inorganique ou organique est présent, le substrat possédant lui-même des propriétés d'acide de Lewis ou de Brônsted ou en appliquant sur le substrat des groupes fonctionnels correspondant d'acide de Lewis ou de Brönsted, et l'application de ces groupes étant réalisée de façon chimique ou physique.

7. Procédé selon la revendication 6,
**caractérisé en ce que**
comme substrat on utilise un solide inorganique acide à base d'oxyde d'aluminium ou d'aluminosilicate.

8. Procédé selon l'une quelconque des revendications précédentes,
**caractérisé en ce que**
comme substrat on utilise un solide organique à base d'échangeurs d'ions acides ou fortement acides.

9. Procédé selon l'une quelconque des revendications précédentes,
**caractérisé en ce que**
le rapport en poids du catalyseur par rapport au substrat et de 0,1 : 1 à 10 : 1.

10. Procédé selon l'une des revendications 1 à 9,
**caractérisé en ce qu'**
on utilise un catalyseur ou un catalyseur et un substrat sous forme pulvérulente.

11. Procédé selon l'une quelconque des revendications précédentes,
**caractérisé en ce qu'**
un catalyseur et un substrat sont utilisés sous forme moulée.

12. Procédé selon l'une quelconque des revendications précédentes,
**caractérisé en ce qu'**
on utilise un corps moulé en fondant le substrat en même temps comme liant du corps moulé.

13. Procédé selon l'une quelconque des revendications précédentes,
**caractérise en ce que**
le catalyseur hétérogène est sous forme d'un lit fixe.

14. Procédé selon l'une quelconque des revendications précédentes,
**caractérisé en ce que**
l'anion du sel d'ammonium est un ion d'halogénure, de sulfate, d'hydrogénosulfate, de nitrate ou de phosphate ou un anion d'un acide carbonique.

15. Procédé selon la revendication 13,
**caractérisé en ce que**
comme sel d'ammonium sont présents du formiate, de l'acétate, du propionate, de l'oxalate, du glutyrate, du malonate, du citrate ou du benzoate d'ammonium.

16. Procédé selon l'une quelconque des revendications précédentes,
**caractérisé en ce que**
la température de réaction s'inscrit entre 20°C et 150°C.

17. Procédé selon la revendication 16,
**caractérisé en ce que**
la température de réaction s'inscrit entre 60°C et 100°C.

18. Procédé selon l'une quelconque des revendications précédentes,
**caractérisé en ce que**
la pression durant la réaction s'élève entre 1 bar et 10 bars.

19. Procédé selon l'une quelconque des revendications précédentes,
**caractérisé en ce que**
comme composé carbonyle, on ammoxime l'acétophénone ou les cétones cycliques avec 8 à 20 atomes de carbone.

20. Procédé selon la revendication 18,
**caractérisé en ce qu'**
on ammoxime de la cyclododécanone.
